# EUROPEAN PATENT SPECIFICATION

(11) **EP 4 226 900 B1**
(45) Date of publication and mention of the grant of the patent: **23.10.2024**
(21) Application number: 23159130.6
(22) Date of filing: 12.07.2016
(51) Int. Cl.: A61F 5/08

(54) **NASAL DILATOR DEVICE**
NASALE DILATATORVORRICHTUNG
DISPOSITIF DILATATEUR NASAL

(30) Priority: 13.07.2015 IT UB20152122
(43) Date of publication of application: 16.08.2023
(62) Divisional of application: 16179015.9
(73) Proprietor: EUPRANA S.R.L., 10128 Torino (IT)
(72) Inventor: AVATANEO, Roberto, 10122 Torino (IT)
(74) Representative: Studio Torta S.p.A.

(56) References cited:
- US-A- 5 669 377
- US-A- 5 961 537
- US-A1- 2010 326 448
- US-A1- 2011 106 140

## Description

The present invention relates to a nasal dilator device of the external type, that is to say one that is applicable to the outer surface of the nasal wall to increase the flow cross section of the air inhaled during breathing.

Nasal dilators of the external type include known dilators generally referred to as nasal patches or strips, such as those described in WO 94/23675 and WO 93/22340.

This type of dilator has a structure similar to that of an adhesive plaster, usually including two elastic strip-like elements, and is intended to be applied transversely to the nasal bridge, in a curved configuration, with the end portions adhering to the outer surface of the walls of both nostrils; the aforesaid elastic elements, which tend to return to the initial flat configuration, generate a tension on the walls of both nostrils which tends to keep the anterior nasal apertures open, thus preventing their collapse and increasing the flow cross section of air in the nostrils.

Following appropriate application, this type of dilator is considered to facilitate breathing, and it is therefore used in sporting activities, as an aid for reducing congestion, and for the prevention of snoring.

A nasal dilator in accordance with the preamble of claim 1 is known from US 5,961,537 A, which is also considered to represent the closest prior art.

The object of the present invention is to provide an alternative external dilator which has non-invasive characteristics for the person wearing it and which is comfortable in use, while also acting effectively to increase the flow cross section of the inhaled air.

In view of this object, the invention proposes a nasal dilator device having the characteristics defined in the claims below, which are to be considered an integral part of the present description.

Further characteristics and advantages of the nasal dilator device according to the invention will be apparent from the following detailed description, which illustrates currently preferred embodiments of the device, provided purely by way of non-limiting example.

In the attached drawings,
- Figure 1 is a perspective view of an exemplary device not falling under the scope of the present invention, applied to the user's nose;
- Figure 2 is a side view of the dilator device applied as in Figure 1; and
- Figure 3 is a perspective view of a dilator device according to the invention, applied to the user's nose.

A common characteristic of the dilator devices according to the invention is that these devices can increase the flow cross section of the air inhaled during breathing by an action of lifting the nasal tip.

In particular, the nasal dilator device according to the invention is characterized by a structure comprising a shaped strip-like body adapted to engage the columella and equipped with adhesive fastening means, connected to said strip-like body to adhere to regions of the nasal wall on opposite sides of the nasal bridge so as to exert a lifting action on the nasal tip by means of said strip-like body.

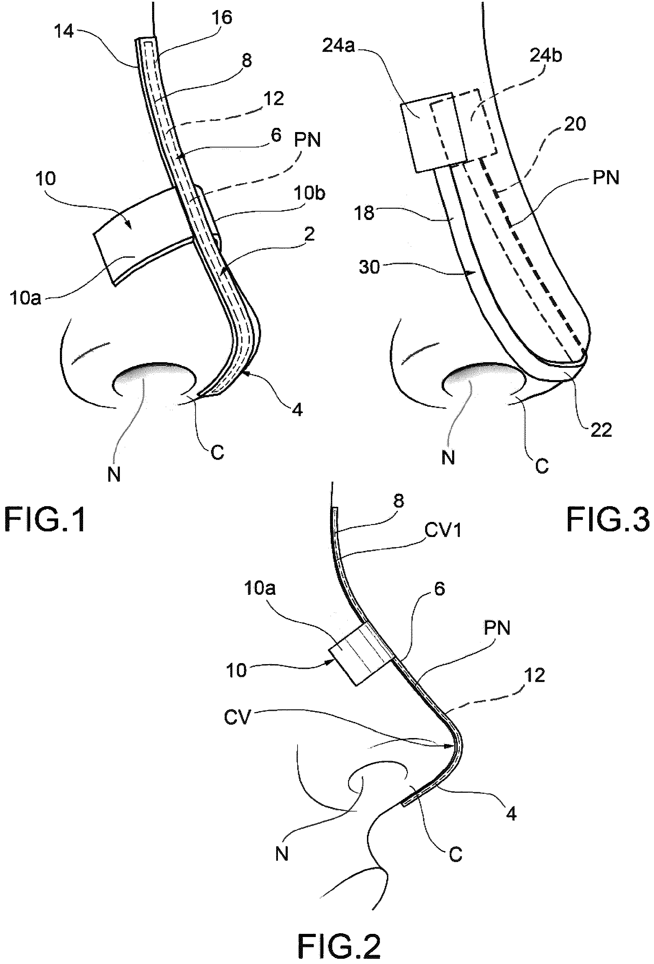

In Figures 1 and 2, the dilator device comprises an elongate flattened body 2 including a branch 4, defined here as descending, and a branch 6, defined here as ascending, connected to one another with a curvature CV substantially complementary to the curvature of the profile of the junction between the columella C and the nasal bridge PN. The aforesaid ascending branch 6 is dimensioned to extend along the nasal bridge PN, and the aforesaid descending branch 4 is dimensioned to extend along at least a portion of the columella.

In an embodiment, the aforesaid ascending branch 6 is also dimensioned to extend along a portion of the profile of the nasofrontal angle, thus having a terminal portion 8 which is connected to the generally rectilinear portion extending along the nasal bridge with a curvature CV1 substantially complementary to the curvature of the nasofrontal angle.

The shaped body 2 is associated with adhesive fastening means 10, positioned to adhere to regions of the nasal wall on opposite sides of the nasal bridge PN; said adhesive fastening means 10, in particular, are connected to the shaped body in a region of the ascending branch 6 in the region above the nostrils, at the position of the nasal bones. In the illustrated configuration, the fastening means comprise a first and a second tab 10a, 10b, having an adhesive surface facing the surfaces of the nasal walls. These tabs are designed to be configured and structured in a manner which is known for adhesive strips; that is to say, they are, for example, structured in the form of breathable fabric tabs with an adhesive lower surface and a removable protective liner applied to the adhesive face.

In an embodiment, which enables the user to wear the device more comfortably, the body 2 has a structure with an inner core 12 (indicated by broken lines in Figures 1 and 2), formed by a shaped strip or flat piece as indicated above for the body 2, incorporated between a first and a second covering 14 and 16, made of sheet material such as a breathable fabric. The core 12 may be fastened to the coverings 14 and 16 by means of adhesive; optionally, it may have a single covering, preferably adhering to the side of the core facing the surface of the nose. The adhesive means 10 may possibly be interposed between the two coverings 14 and 16 or may be integral with one of these coverings.

The material used for the shaped body or for the core is a material which is elastic and therefore flexible, but which is stiff enough not to be deformed under the action of the forces to which it is subjected in use, in order to ensure that the aforementioned curvatures CV and CV1 are not altered during use.

The device is worn as shown in Figures 1 and 2, care being taken to apply the adhesive fastening means 10 in such a position that the device, having been fastened, can exert the aforesaid action of lifting the nasal tip.

In the embodiment according to the present invention of Figure 3, the dilator device has a body shaped in a loop configuration, with a first branch 18 and a second branch 20 (the latter being indicated by broken lines in Figure 3) connected to one another in a U-shape, wherein the connecting portion 22 between the two branches is curved so as to engage the columella under the nasal tip.

Each branch 18 and 20 is dimensioned to extend along a respective side of the nasal wall, on opposite sides of the nasal bridge, and has at its upper end adhesive fastening means 24a and 24b, connected to said end and capable of being made to adhere to the nasal bridge.

The adhesive fastening means are configured in a known way, having an adhesive lower surface, provided if necessary with a protective liner to be removed by the user, and an upper surface of breathable fabric or plastic material.

In this case also, the shaped body 30 of the dilator may have a structure, as indicated with reference to Figures 1 and 2, that is to say a structure with an inner core (not shown) and a first and/or a second covering. The core, as mentioned above, is usually a strip or flat piece of metal or plastic material. The dilator may be applied by the user, care being taken to position the adhesive means 24a and 24b in such a way that the shaped body 30 exerts a lifting action on the nasal tip, resulting in dilation of the nostrils.

In all its embodiments, the dilator device may be made in different sizes or with different measurements, to be adaptable to the user, who may be an adult male or female or a child.

In the embodiment of Figures 1 and 2, auxiliary fastening means of the type indicated by 10 may also be present, being connected to the shaped body, for example immediately under the nasofrontal angle.

The device is suitable for use in all known applications for nasal dilators of the external type, for the purpose of improving breathing, preventing congestion phenomena and reducing the incidence of snoring and apnoea.

In the embodiment shown in Figure 3, the device may also be used in post-surgical treatment for rhinoplasty operations for lifting the nasal tip.

Clearly, provided that the principle of the invention is retained, the forms of application and the details of embodiment can be varied widely from what has been described and illustrated by way of non-limiting example, without departure from the scope of the claims below.

## Claims

1. A nasal dilator device applicable to the outer surface of the nasal wall to increase the flow cross section of the air inhaled during breathing, comprising a shaped flattened body (30) adapted to engage the columella (C) and adhesive fastening means (24a, 24b), connected to the flattened body to adhere to regions of the nasal wall on opposite sides of the nasal bridge (PN) so as to exert a lifting action on the nasal tip by means of said flattened body **characterized in that** said flattened body comprises a loop-shaped flattened body (30) having a first (18) and a second (20) branch, said first and second branch (18, 20) being connected in a U-shape by means of a connecting portion (22) adapted to engage the columella (C), and said fastening adhesive means (24a, 24b) are connected to the terminal ends of respective branches (18, 20).

2. Dilator device according to Claim 1, **characterized in that** said loop-shaped body comprises a core (12) incorporated between a first and a second covering (14, 16).

## Patentansprüche

1. Nasendilatatorvorrichtung, die an der Außenfläche der Nasenwand anbringbar ist, um den Strömungsquerschnitt der während der Atmung eingeatmeten Luft zu vergrößern, umfassend einen geformten abgeflachten Körper (30), der so ausgebildet ist, dass er an der Columella (C) angreift, und klebende Befestigungsmittel (24a, 24b), die mit dem abgeflachten Körper verbunden sind, um an Bereichen der Nasenwand auf gegenüberliegenden Seiten der Nasenbrücke (PN) zu haften, um so eine Hebewirkung auf die Nasenspitze mittels des abgeflachten Körpers auszuüben, **dadurch gekennzeichnet, dass** der abgeflachte Körper einen schleifenförmigen abgeflachten Körper (30) mit einem ersten (18) und einem zweiten (20) Zweig umfasst, der erste und der zweite Zweig (18, 20) mittels eines Verbindungsabschnitts (22), der zum Eingriff in die Columella (C) ausgebildet ist, U-förmig verbunden sind und die klebenden Befestigungsmittel (24a, 24b) mit den abschließenden Enden der jeweiligen Zweige (18, 20) verbunden sind.

2. Dilatatorvorrichtung nach Anspruch 1, **dadurch gekennzeichnet, dass** der schleifenförmige Körper einen Kern (12) umfasst, der zwischen einer ersten und einer zweiten Abdeckung (14, 16) ausgebildet ist.

## Revendications

1. Dispositif dilateur nasal applicable sur la surface extérieure de la paroi nasale pour augmenter la section de débit de l'air inhalé au cours de la respiration,
comprenant :
un corps de forme aplatie (30) adapté pour s'engager sur la columelle (C) et un moyen de fixation par adhésif (24a, 24b) relié au corps aplati pour s'adhérer aux régions de la paroi nasale sur les côtés opposés de l'arête nasale (PN) pour exercer une action de soulèvement sur l'extrémité nasale par le corps aplati,
dispositif **caractérisé en ce que**
le corps aplati est un corps aplati en forme de boucle (30) ayant une première branche (18) et une seconde branche (20), la première et la seconde branche (18, 20) étant reliées en forme de U par une partie de connexion (22) adaptée pour s'engager sur la columelle (C) et les moyens de fixation par adhésif (24a, 24b) étant reliés à l'extrémité terminale des branches respectives (18, 20).

2. Dispositif dilateur selon la revendication 1,
**caractérisé en ce que**
le corps en forme de boucle comprend un coeur (12) incorporé entre une première et une seconde couvertures (14, 16).
